# EUROPEAN PATENT SPECIFICATION

(11) **EP 2 277 990 B1**
(45) Date of publication and mention of the grant of the patent: **02.07.2014**
(21) Application number: 10170494.8
(22) Date of filing: 22.07.2010
(51) Int. Cl.: C12N 1/18, C12G 1/022, C12G 3/02, C12C 11/00, C12R 1/865

(54) **Strain of CECT 13030 of Saccharomyces cerevisiae and its use for the production of alcoholic beverages**
Saccharomyces cerevisiae-Stamm CECT 13030 und dessen Verwendung zur Herstellung von alkoholischen Getränken
Souche de Saccharomyces cerevisiae CECT 13030 et son utlisation pour la production de boissons alcoolisées

(30) Priority: 22.07.2009 ES 200901625
(43) Date of publication of application: 26.01.2011
(73) Proprietor: Guserbiot S.L.U., 01015 Vitoria Alava (ES)
(72) Inventor: Barbero Mangas, Francisca, 01015 Vitoria (ES); Abadin Insua, Cristina, 01015 Vitoria (ES); Oyanguren García, Iñigo, 01015 Vitoria (ES); Zumárraga Uribesalgo, Miren, 01015 Vitoria (ES)
(74) Representative: Pons Ariño, Angel

(56) References cited:
- EP-A1- 1 482 029
- DE-A1- 3 701 193
- ES-A1- 2 091 723
- ES-A1- 2 334 753
- GUTIERREZ A R ET AL: "Killer yeasts: Incidence in the ecology of spontaneous fermentation" AMERICAN JOURNAL OF ENOLOGY AND VITICULTURE, vol. 52, no. 4, 2001, pages 352-356, XP008127650 ISSN: 0002-9254
- GUTIERREZ ANA ROSA ET AL: "Ecology of inoculated and spontaneous fermentations in Rioja (Spain) musts, examined by mitochondrial DNA restriction analysis" INTERNATIONAL JOURNAL OF FOOD MICROBIOLOGY, vol. 36, no. 2-3, 1997, pages 241-245, XP002603387 ISSN: 0168-1605
- NIKOLAOU E ET AL: "Selection of indigenous Saccharomyces cerevisiae strains according to their oenological characteristics and vinification results" FOOD MICROBIOLOGYS, vol. 23, no. 2, 1 April 2006 (2006-04-01), pages 205-211, XP024904302 ISSN: 0740-0020 [retrieved on 2006-04-01]
- CHRISTIAN A LOPES ET AL: "Patagonian wines: the selection of an indigenous yeast starter" JOURNAL OF INDUSTRIAL MICROBIOLOGY & BIOTECHNOLOGY, vol. 34, no. 8, 19 June 2007 (2007-06-19), pages 539-546, XP019521823 ISSN: 1476-5535
- TRONCHONI J ET AL: "Differences in the glucose and fructose consumption profiles in diverse Saccharomyces wine species and their hybrids during grape juice fermentation" INTERNATIONAL JOURNAL OF FOOD MICROBIOLOGY, vol. 134, no. 3, 12 July 2009 (2009-07-12) , pages 237-243, XP026495592 ISSN: 0168-1605 [retrieved on 2009-07-12]
- INAKI ETAIO ET AL: "Sensory attribute evolution in bottled young red wines from Rioja Alavesa" EUROPEAN FOOD RESEARCH AND TECHNOLOGY, vol. 228, no. 5, 11 November 2008 (2008-11-11), pages 695-705, XP019702906 ISSN: 1438-2385

## Description

In the course of grape must fermentation, the sequential action of different genii and species of yeast occurs. In the first stages of fermentation (4-5% vo. alcohol) indigenous yeasts are developed with low fermentation power of the species: *Kloeckera, Hanseniaspora, Candida* and *Pichia.* Once 4-5% alcohol has been exceeded, the fermentation process is fundamentally dominated by the species *Saccharomyces cerevisiae,* more resistant to alcohol.

The origin of these yeasts is fundamentally on the surface of the grapes as well as the environment of the winery (machinery, hoppers, presses, fermentation tanks, etc.), this latter being an important source of microorganisms that inoculate the grape musts. The number of yeasts present in the skin of a healthy grape is very low (between 10³ and 10⁶ CFU/mL) and the number of *Saccharomyces cerevisiae* is even less, less than 50 cells/mL of grape must. In the winery, *S. Cerevisiae* is the predominant species.

The microflora present in the grape can be affected by a large number of factors that influence the number and proportion of the different species.

These factors include the temperature, rainfall and other climatic influences, the degree of ripeness of the grape, the use of phytosanitary treatments, physical damage due to fungi, insects, etc. and the grape variety. Variations in the initial flora may influence the fermentation process, causing delays and even stoppages of fermentation, and may affect the quality of wine and give rises to changes in volatile acidity and unpleasant tastes and/or aromas.

Indeed, variability of the yeast-like flora of the grape musts can be resolved by adding in each grape harvest a microbial inoculation which on being majority normalizes the initial flora and, in this way, gives rise to a homogeneous fermentation in the winery year after year.

When selecting the yeasts for the production of quality wines, it is sought that the yeasts have the following characteristics:
- High ethanol tolerance
- Total degradation of fermentable sugars
- Resistance to SO₂
- High fermentation capacity
- Glycerol production
- *Killer* phenotype

In contrast, it is attempted to avoid that the yeast has the following undesired qualities:
- H₂S production
- Volatile acidity production
- Production of acetaldehyde and pyruvate
- Formation of ethyl carbamate precursors

At present, almost all wineries used commercial yeast inoculations for fermenting their grape musts. The use of these commercial inoculations in wineries reduces the risk of slow fermentation kinetics and defects in the taste and aroma of wine. Due to this, the inoculation of the must with these yeasts is a widespread practice in wineries. These yeasts have mainly been isolated in areas, grape varieties and conditions other than those existing in the wineries which also use them equally in other Spanish designations of origin and from other countries, the consequence of their use being the production of wines with a certain loss of typical characteristics.

Against this drawback of a widespread application and loss of typical characteristics, an alternative that is demonstrating its validity is the use of native yeasts, isolated from grapes in a certain area to make wines from that same area. In this way it is possible to control the fermentation process also respecting the local character of the wines. This point should therefore indicate the numerous advantages that the use of native yeasts would have when they are selected in the designations of origin for the winemaking. On the other hand, the selection should be centred on the grapes, not in the winery environment, as wineries precisely have a residual population of yeasts of varied origin and largely commercial.

### DESCRIPTION OF THE INVENTION

In a first aspect, the present invention provides a strain of the species *Saccharomyces cerevisiae* identified as GBiot-EL 011, said strain was deposited on 12 May 2009 in the Spanish Type Culture Collection (CECT), Burjasot, Valencia, with access number CECT 13030.

In a more particular aspect, the strain of the present invention is in liquid form, fresh in paste, active dry and instant dry.

In a second aspect, the present invention relates to the use of the strain of the species *Saccharomyces cerevisiae* identified as GBiot-EL 011 deposited in the CECT, with access number CECT 13030 in the preparation of alcoholic drinks produced by alcoholic fermentation.

In a more particular aspect, the alcoholic drink is selected from wine, beer, cava and cider.

In another more particular aspect, the alcoholic drink is wine produced from grape musts. In another more particular aspect, the musts are from grapes from the Rioja Alavesa wine region, more in particular they are grape musts from the red wine variety, more in particular they are of the variety Garnacha, Graciano, Mencia, Monastrell, Mazuelo, Bobal, more in particular the grape is of the Tempranillo variety.

In a third aspect, the present invention relates to the use of the strain of the species *Saccharomyces cerevisiae* identified as GBiot-EL 011 deposited in the CECT, with access number CECT 13030 for biomass production.

### DESCRIPTION OF THE FIGURES

Figure 1: Genetic profiles of the strain GBiot-EL 11 (CECT 13030) obtained by electrophoretic separation in agarose gels using the inter delta sequence amplification technique (A) and the analysis of mitochondrial DNA restriction fragments (B). M: molecular weight marker (phage λ digested with restriction enzyme *Pstl.*)
Figure 2: Fermentation kinetics of the native yeast and the commercial yeast in microvinification I of example 2. The fermentation kinetics of these yeasts is expressed as the evolution of the °Brix of the grape must over time.
Figure 3: Fermentation kinetics of the native yeast and the commercial yeast in microvinification III of example 2.
Figure 4: Fermentation kinetics of the native yeast in formats: fresh in paste, dry and of the commercial yeast in microvinification I of example 3.
Figure 5: Fermentation kinetics of the native yeast in formats: fresh in paste, dry and of the commercial yeast in microvinification II of example 3.
Figure 6: Fermentation kinetics of the native yeast in formats: fresh in paste, dry and of the commercial yeast in microvinification III of example 3.
Figure 7: Fermentation kinetics of the native yeast in formats: fresh in paste, dry and of the commercial yeast in microvinification IV of example 3.
Figure 8: Fermentation kinetics of the native yeast and the commercial yeast in microvinification I of example 5.
Figure 9: Fermentation kinetics of the native yeast and the commercial yeast in microvinification II of example 5.

### DESCRIPTION OF THE INVENTION

The stain GBiot-EL 011 deposited in the Spanish Type Culture Collection (CECT) with access number CECT 13030, has the following characteristics:

### Morphology:

- Cellular morphology: oval, rounded cells
- In solid medium (malt extract agar) the colony had a whitish colour, smooth and shiny surface.
- In lysine agar it showed no growth.

### Metabolic characteristics

- Fermentation of carbohydrates in aerobiotic conditions, the strain was: Glucose (+), maltose (+), sucrose (+), trehalose (+), raffinose (+), α glucosidase (+), xylose (+), galactose (+), histidine (+), leucyl-glycine (+), proline (-) and lactose (-).
- Fermentation of carbohydrates in anaerobiotic conditions, the strain was: Glucose (+), maltose (+), sucrose (+), trehalose (+), raffinose (+), β glucosidase (+) and galactose (+).
- Assimilation of nitrogenated compounds, the stain was: nitrate (-), lysine (-).
- Oxygen requirements: it is facultative anaerobic.
- Growth at different temperatures: the strain grew, at least, in a temperature range of between 14°C and 37°C.
- Resistance to cycloheximide: it showed its growth limit in the presence of 10 ppms of cycloheximide.

### Enological/technical characteristics of the strain:

The strain GBiot-EL 011 (CECT 13030) showed fast and regular fermentation kinetics with a short latency phase. It was capable of performing alcoholic fermentations within a wide temperature range, at least between 14°C and 30°C, however it was optimum for the fermentation of red wines with a temperature range of 20-30°C. The flocculation capacity was good and with average foam formation.

The alcohol yield was high: 16.8 grams of sugar per degree of alcohol produced. It almost completely exhausted all reducing sugars present in the grape must, leaving the wine practically dry (between 1-6 g/L residual sugars).

The strain GBiot-EL 011 (CECT 13030) showed high resistance to sulphur dioxide, resisting concentrations of up to 100 g/L of total SO₂. The yeast strain GBiot-EL 011 (CECT 13030) was inoculated in a culture medium, which contained concentrations of 50, 75 and 100 mg/L of SO₂ and it was observed if growth occurred after 48-72 hours of incubation.

The ethanol tolerance was also high, around 16% vol. Ethanol resistance was determined by inoculation of a native strain both in the liquid culture medium and in Petri dishes with ethanol content of 12, 14 and 6% v/v.

The strain GBiot-EL 011 (CECT 13030) has a neutral *Killer* phenotype, in other words, it does not secrete the *Killer* toxin but it is resistant to it. Determination of the *Killer* phenotype is based on inoculating the yeast to be tested in a dish with a buffered and inoculated medium with a lawn of sensitive yeast, and on another dish, with the same medium inoculated with a lawn of *Killer* yeast (Bevan and Somers, 1969). After its incubation at 27°C during 72 h growth was observed on both dishes on the two lawns of control strains without the appearance of inhibiting halos.

Hydrogen sulphide production was low. The method used to determine H₂S formation was based on the degree of darkening of the colonies growth in a Biggy (Bismuth-Glucose-Glycine-Yeast) agar medium, a modification of the formula developed by Nickerson (1953). This medium contains bismuth sulphite and varies from cream colour (unproductive strains) to dark brown (very productive strains). The degree of darkening is determined by bismuth sulphide, which is formed by the combination of bismuth in the medium and H₂S released by the yeast.

Glycerol production was average, 8 g/L of glycerol. The glycerol produced during the alcoholic fermentation was determined by the enzymatic method developed by Kreutz (1962) modified.

Volatile acidity production was also low (<0.54 g /L of acetic acid). The García-Tena method was used (García Barceló, 1976).

### Generic characterization

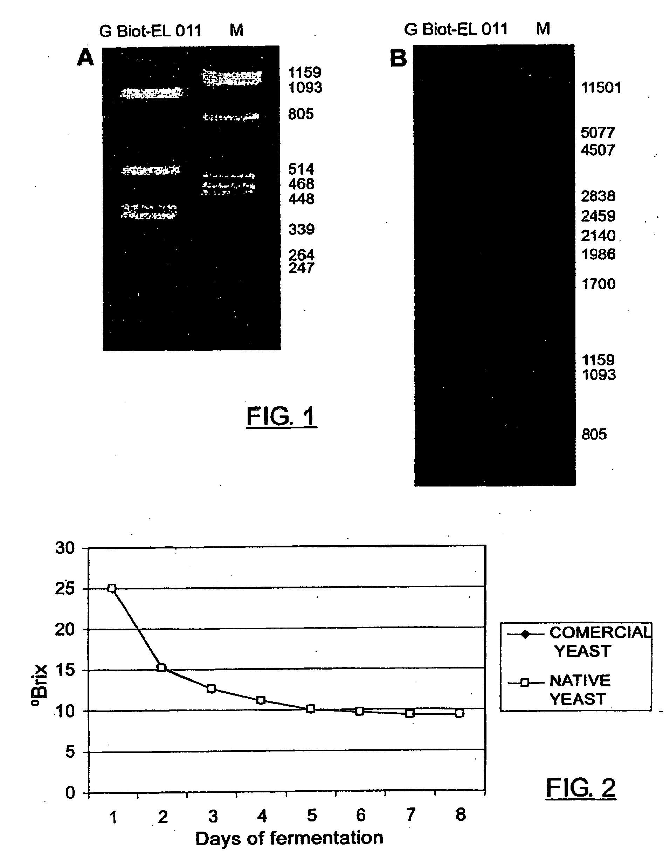

The genetic characterization of the strain GBiot-EL 011 (CECT 13030) was obtained by two complementary molecular techniques: PCR (polymerase chain reaction) amplification of the inter delta sequences that flank the Ty1 retrotransposons (Ness, 1993) and the analysis of the polymorphisms of the mitochondrial DNA restriction fragments (Querol, 1992). Figure 1 shows the genetic profiles of the strain GBiot-EL 011 (CECT 13030) obtained with each of the techniques.

### Biomass production

Biomass production was carried out by Fed-Batch culture, in a fermenter with a total capacity of 12 litres (Biostat C), with continuous control of pH, temperature, dissolved oxygen and agitation. The feeding was carried out using a food medium with a total sugar concentration of 250 g/L (sucrose, glucose, fructose), supplemented with a source of nitrogen, magnesium, vitamins (biotin, inositol and calcium pantothenate) and mineral salts (iron sulphate, zinc sulphate and copper sulphate).

The fermenter was inoculated with an inoculation volume guaranteeing a cell count in the initial fermentation volume of 10⁸ CFU/mL.

The fermentation conditions were as follows:
- pH: the pH of the culture was kept at 4.5 throughout the process by adding 25% NaOH (w·v⁻¹) and 75% H₃PO₄ (w·v⁻¹)
- Temperature: 30°C
- Agitation: 700 rpm

The biomass production process lasted in the order of 40 hours. The feasibility obtained by mL of the fermented broth reached the order of 10⁹ CFU/mL.

We obtained the following formats for the yeast from this broth:
- Liquid format: fermented broth with no type of treatment with a viable cell count ≥ 10⁹ CFU/mL.
- Paste format: obtained from the filtration, by press filter, of the fermented broth. This treatment allowed us to obtain a cream with a viable cell count ≥ 10¹⁰ CFU/g.
- Active dry format: this format was obtained from the yeast paste and with a dehydration treatment consisting of fluid bed drying. The product must have a humidity content less than 8%. The drying at 37°C for 60 minutes provided a dry yeast product with a viable cell count ≥ 5 x 10⁵ CFU/g. The product must be previously rehydrated for its application.
- Instant dry formula: this format was obtained from the yeast paste and with a dehydration treatment consisting of fluid bed drying. The product must have a humidity content less than 5% and a viable cell count ≥ 10¹⁰ CFU/g.

### Example 1. Obtaining the strain GBiot-EL 011(CECT 13030)

The isolation and selection of the yeast was carried out during two consecutive wine harvests. As a result of this study, a native yeast strain was selected of the species *S. cerevisiae,* GBiot-EL 011 (CECT 13030). The testing of the native yeast selected was performed during 3 wine harvests.

For the isolation of native yeasts, healthy and ripe grapes of the Tempranillo variety were collected in several vineyards belonging to the Rioja Alavesa wine region. Samples were taken from young vines (stock with age less than 25 years), old vines (stock with age between 25-50 years) and native vines (stock with an age over 50 years).

The grapes were transferred to the laboratory and were stored in a fresh and aerated place until their processing. At this time, they were pressed and fermented in sterile receptacles. Said receptacles were introduced in an orbital incubator with controlled temperature of 27°C. Throughout the fermentation, monitoring was performed on the °Brix and once they were stabilized, these containers were sampled to isolate the yeasts. The samples obtained to isolate the yeasts were inoculated, after the corresponding decimal dilutions, on Petri dishes containing malt agar extract and they were left to incubate at 27°C. From each dish, with 30-300 colonies, three different colonies were taken with an appearance in line with *Saccharomyces cerevisiae.* The total yeast strains isolated from the grape, with appearance of *S. cerevisiae* was 175.

Once all the yeasts were characterized, those that complied with the following technological characteristics were selected:
- Growth on lysine-agar: negative
- Rapid test: *S. cerevisiae*
- *Killer* phenotype: neutral or *Killer*
- Formation of hydrogen sulphide: medium or low
- Sulphur dioxide resistance: positive growth at least on dishes with 50 mg/L SO₂/L
- Ethanol resistance: positive growth at least on dishes with 12% ethanol.

Of all the yeasts isolated, 76 of the strains were identified as *S. cerevisiae* using the Rapid Yeast Plus System (Remel).

The total of yeasts selected under these criteria were 41.These yeasts were subjected to a first microvinification process with sterile grape must.

The microvinifications consisted of the inoculation with the native yeasts characterized in a sterile grape must and later vinification on laboratory scale under controlled conditions. Inoculation with preselected native yeasts were performed in grape must of the Tempranillo variety from Rioja Alavesa,

This microvinification process allowed us to reject the yeasts with unsuitable kinetic fermentations and which gave rise to unsuitable yeasts from the organoleptic standpoint.

The fermentation process was carried out in 250 mL glass fermentation flasks, in duplicate, immersed in a thermostatic bath with agitation and controlled temperature of 16 to 27 °C, depending on the product one wants to obtain. The yeasts to test were inoculated in a proportion which allowed us to reach in all cases the initial population of 10⁶ cells/mL. The fermentation process was followed by daily measurement of °Brix until reaching a constant value. At that time the wine was decanted, centrifuged and stored in inert nitrogen atmosphere or it was sulphited (20 mg SO₂/L) until the time of wine tasting. The following aspects were assessed in the wine tasting:
- Colour: the formation of robe, limpidity and shine was assessed.
- Nose: intensity, complexity and with aromas without defects.
- Palate: tannins and body

In addition to the tasting, an analysis of the following parameters of the wines produced was also performed: pH, sugars, degree, free sulphur, combined sulphur and total sulphur, volatile acidity, total acidity, glycerol, malic acid, colour intensity, tonality, total polyphenol index, total anthocyanins, total tannins and CIELAB parameters. These analyses were performed by FUNDACION LEIA.

The results obtained in this vinification process allowed us to reject all those strains without good fermentation activity and which gave rise to organoleptic defects in the wine.

The strains with good activity and fermentation kinetics and which were positively valued at tasting, were identified by molecular biology tests (PCR and RFLP of mitochondrial DNA). This molecular characterization revealed to us how many of the strains isolated and identified as *S. cerevisiae* were genetically different, in other words, were different strains.

With the information obtained in the microvinification, both of fermentation kinetics, results of tasting and wine analysis and with the molecular characterization information of the previously selected strains of said microvinification, another two microvinifications were performed which selected for us the strains which we tested on an industrial scale, i.e. in the winery. These microvinifications tested the strains varying the conditions of the grape must and depending on the application sought.

To select the yeast strains for the alcoholic fermentation to obtain the red wine, the following microvinifications were performed:
Microvinification of sugar-rich grape must: the grape must used as substrate, had a sugar content of 25 °Brix, which gave 14.8° proof.
Microvinification of grape must with paste: it was a microvinification where the grape must contained a great amount of material in suspension such as lees, skins, etc. which simulated the conditions of the grape must in the winery.

In the case of selection of strains for refermentation processes, in addition to testing these conditions, other microvinifications were tested such as:
- Microvinification of grape must with low nutrient content, such as nitrogen and vitamins.
- Microvinification at low temperature 12°C and high temperature 33°C
- Microvinification with inactive grape must, with sugar and high alcohol content.
   Depending on the fermentation kinetics, the analytical results obtained and the tasting results, the strains tested in the winery were selected.

### Example 2. Microvinification with different musts and liquid yeast

The microvinification was performed on two different Tempranillo grape musts which had the physicochemical characteristics described in table 1.

**Table 1. Analytical parameters of the starting grape musts for the microvinifications.**

| ANALYSIS | MUST 1 | MUST 2 |
|---|---|---|
| pH | 3.83 | 3.65 |
| °Brix | 22 | 23.4 |
| Density (g/ml) | 1093 | 1098 |
| Reducing sugars (g/L) | 190 | 208 |
| Probable degree (%v/v) | 12.8 | 13.7 |
| YAN (mg/L) | 167 | 149 |
| Free SO₂ (mg/L) | 0 | 13 |
| Total SO₂ (mg/L) | 3 | 26 |
| Volatile acidity (g/L acetic acid) | 0.06 | 0.03 |
| Total acidity (g/L tartaric acid) | 5.2 | 5.7 |
| Malic acid (g/L) | 3.3 | 3.9 |
| Glycerol (g/L) | 0.58 | 0.57 |
| Colour intensity | 5.888 | 4.02 |
| Tonality | 0.699 | 0.43 |
| TPI | 30 | 19 |
| Total anthocyanins (mg/L) | 217 | 278 |
| Total tannins (mg/L) | 1.5 | 1 |
| CIELAB parameters: | | |
| L* significance | 21.219 | 39.085 |
| a* significance | 45.099 | 61.8 |
| b* | 24.936 | 20.127 |

### Microvinification 1

This microvinification compared the native yeast GBiot-EL 11 (CECT 13030) isolated from grapes from the vineyards of Rioja Alavesa together with a commercial yeast Zymaflore F15 which was used as reference. A sterile Tempranillo must was used as substrate, indicated as must 2 in table 1 (sterile must without microbial population and without skins).

Both the native yeast and the commercial strain had fast and regular fermentation kinetics and ended fermentation after 19 days.

The analytical results of the wines obtained from inoculate must are summarized in table 2.

| SAMPLE | COMMERCIAL YEAST | NATIVE YEAST |
|---|---|---|
| pH | 3.46 | 3.43 |
| °Brix | 7.5 | 7.5 |
| Degree (v/v) | 13.5 | 13.62 |
| EtOH yield (%) | 47.4 | 47.8 |
| Reducing sugars (g/L) | 3.1 | 3.6 |
| Free SO₂ (mg/L) | 0 | 0 |
| Combined SO₂ (mg/L) | 9 | 6 |
| Total SO₂ (mg/L) | 9 | 6 |
| Volatile acidity (g/L acetic acid) | 0.26 | 0.29 |
| Total acidity (g/L tartaric acid) | 6.3 | 6.3 |
| Glycerol (g/L) | 8.5 | 8.5 |
| Malic acid (g/L) | 3.08 | 3.4 |
| Colour intensity | 3.557 | 3.3 |
| Tonality | 0.86 | 0.862 |
| TPI | 21 | 19 |
| Anthocyanins (mg/L) | 144 | 153 |
| Total tannins (mg/L) | 1 | 1 |
| CIELAB parameters: | | |
| L* | 34.184 | 37.163 |
| a* | 30.953 | 30.717 |
| b* | 12.716 | 12.121 |

As can be observed, the native yeast selected leads to producing a wine, after alcoholic fermentation, of characteristics very similar to those produced with the commercial yeast. In the two cases, the wines were correct from an organoleptic standpoint, not noting defects. The results obtained in the analyses did not show significant differences between the two yeasts.

### Microvinification II

In this microvinification the must indicated as must 1 in table 1 was used, which is the same of the previous, which was a sterile must without microbial population and without skins.

Figure 2 represents the fermentation kinetics of these yeasts expressed as the evolution of the °Brix of the must over time. The two yeasts ended fermentation after 13 days and showed similar fermentation kinetics.

Table 3 shows the analytical parameters of the wines obtained in this microvinification.

**Table 3. Analytical parameters of the wines produced with microvinification II.**

| SAMPLE | COMMERCIAL YEAST | NATIVE YEAST |
|---|---|---|
| pH | 3.44 | 3.41 |
| °Brix | 7 | 7 |
| Degree (v/v) | 12.97 | 13.78 |
| EtOH yield (%) | 45.5 | 48.4 |
| Reducing sugars (g/L) | 2.6 | 2.6 |
| Volatile acidity (g/L acetic acid) | 0.27 | 0.21 |
| Volatile acidity (g/L tartaric acid) | 5 | 5 |
| Glycerol (g/L) | 8.8 | 8.5 |
| Malic acid (g/L) | 2.76 | 2.72 |
| Colour intensity | 0.496 | 0.478 |
| Tonality | 1.286 | 1.306 |
| TPI | 6 | 6 |
| Anthocyanins (mg/L) | 20 | 22 |
| Total tannins (mg/L) | 0.3 | 0.3 |
| CIELAB parameters: | | |
| L* | 87.886 | 88.46 |
| a* | 8.987 | 9.123 |
| b* | 9.015 | 9.343 |

In the second microvinification, small differences were observed between the native and commercial yeast, which was appreciated in the value of the degree and ethanol yield produced, those obtained with native yeast being slightly superior.

As regards the assessment in tasting, the wine inoculated with the native yeast stood out due to its intensity and roundness.

### Microvinification III

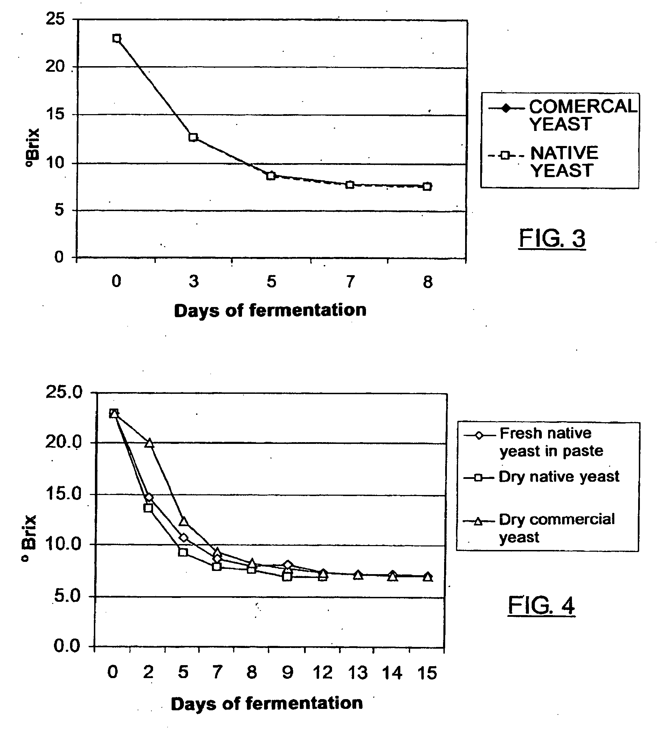

The microvinification performed used as its base the must tested in the first vinification, indicated as must 2, with the skins present in the same must. The fermentation kinetics of the yeast in this must are shown in figure 3.

The fermentations with this must were faster than in the two previous microvinifications, and they were finished in only 8 days. The content of solids in suspension stimulated the growth and metabolism of the yeasts during fermentation. The two yeasts, as in the two previous microvinifications, showed similar kinetics.

The analytical results of the wines obtained in this microvinification are shown in table 4.

**Table 4. Analytical parameters of the wines obtained in microvinification III.**

| SAMPLE | COMMERCIAL YEAST | NATIVE YEAST |
|---|---|---|
| pH | 3.48 | 3.47 |
| °Brix | 7.6 | 7.6 |
| Degree (v/v) | 13.98 | 13.34 |
| EtOH yield (%) | 49.1 | 46.8 |
| Reducing sugars (g/L) | 1.6 | 1.7 |
| Volatile acidity (g/L acetic acid) | 0.24 | 0.21 |
| Volatile acidity (g/L tartaric acid) | 7.2 | 6.5 |
| Glycerol (g/L) | 10 | 9.9 |
| Malic acid (g/L) | 3.56 | 3.54 |
| Colour intensity | 1.981 | 2.123 |
| Tonality | 0.813 | 0.864 |
| TPI | 15 | 15 |
| Anthocyanins (mg/L) | 117 | 112 |
| Total tannins (mg/L) | 0.8 | 0.8 |
| CIELAB parameters: | | |
| L* | 58.139 | 55.992 |
| a* | 45.227 | 42.969 |
| b* | 17.163 | 18.446 |

In this microvinification, small differences were found between the native yeast and the commercial yeast, which was appreciated in the ethanol yield obtained, in this case being slightly higher in the wine fermented with commercial yeast.

With respect to the tasting, the wines were positively assessed, and no defects were appreciated therein.

In addition to the tasting and analysis of the wines, a study of implantation of the two yeasts was carried out, since the must with skins had its own indigenous population. In the two cases, both the native yeast and the commercial yeast were implanted 100%.

### Example 3. Microvinification with different formats of the native yeast.

3 presentation formats of the native yeast were tested in order to assess the fermenting, organoleptic and analytical qualities of the different formulations. The formats were the following: in the form of fresh paste and in the active dry form. The native yeast was compared with Zymaflore F15, frequently used in the preparation of Rioja wine and appreciated by enologists due to its fermentation, analytical and organoleptic characteristics. In the case of the commercial yeast, the test was performed in its active dry form, which is the commercially available format.

As substrates for the fermentations in the laboratory, two grape musts from the Tempranillo variety from Rioja Alavesa were used. The following table includes the main characteristics of these musts.

**Table 5: Physicochemical parameters of the musts used in the microvinifications.**

| **Analytical parameters** | **Must 1** | **Must 2** |
|---|---|---|
| Probable degree (%v/v) | 12.90 | 14.72 |
| Volatile acidity (g/L acetic acid) | 0.03 | 0.03 |
| Total acidity (g/L tartaric acid) | 3.7 | 3.7 |
| Malic acid (g/L) | 2.41 | 2.8 |
| Glycerol (g/L) | 0.19 | 0.25 |
| Colour intensity | 1.994 | 2.472 |
| Tonality | 0.465 | 0.532 |
| TPI | 15 | 16 |
| Total anthocyanins (mg/L) | 140 | 156 |
| Total tannins (mg/L) | 1 | 1 |
| CIELAB parameters: | | |
| L* | 58.153 | 48.673 |
| a* | 57.331 | 49.962 |
| b* | 8.449 | 5.453 |

The microvinifications performed were as follows:
- Microvinification I: sterile must 1, adjusted to 23 °Brix, 257 mg assimilable N/L, 40 mg SO₂/L and pH 3.51
- Microvinification II: sterile must 2 with excess sugars, at 25 °Brix, 260 mg assimilable N/L, 40 mg SO₂/L and pH 3.54
- Microvinification III: must 2 with paste (skins), at 24.6 °Brix, 40 mg SO₂/L and pH 3.42
- Microvinification II: sterile must 1, adjusted to 23 °Brix, 250 mg assimilable N/L, 40 mg SO₂/L and pH 3.5.

The inoculations were prepared as follows:
- Fresh yeast: one aliquot of must was dissolved and directly added.
- Dry yeast: it was dissolved in distilled water with glucose (1:10 w/v), it was incubated at 40 °C for 20 minutes, mixed with one aliquot of must, incubated during 10 minutes and the must was added to start the fermentation.

At this time of inoculating, in all cases an initial population of yeasts of 10⁶ cells/mL was obtained. The fermentations were carried out in 250 mL glass flasks immersed in thermostatted baths with agitation and controlled temperature (27°C). The process was followed by daily measurement of °Brix and it as considered finalized when this was stabilized. The wines produced were decanted, centrifuged and stored in inert nitrogen atmosphere and were sulphited (20 mg SO₂/L) until the time of wine tasting.

Once the microvinifications were finished, the tasting of the wines obtained were performed, assessing their colour, aroma and taste. The corresponding analyses were also performed.

On these data a two-way variance analysis (ANOVA) was carried out (yeast type and format) to detect significant differences between the microvinifications prepared with different yeasts in its different formats. The significance level in all cases is 95%.

### Microvinification I

Must 1 was used in this microvinification as substrate for fermentation.

Figure 4 shows the fermentation kinetics of these yeasts expressed as the evolution of the °Brix of the must over time.

As observed in Figure 4, the native yeast (fresh or dry) started fermenting rapidly, whilst the commercial yeast started a little later. All fermentations ended in a similar time and with similar °Brix.

The analytical results of the wines obtained from the microvinifications of the native yeast and the control yeast in its different formats are shown in table 6.

**Table 6: Physicochemical parameters of the wines obtained by inoculation of the native yeast in its different formats and the commercial yeast on must 1.**

| | | | |
|---|---|---|---|
| Yeast | NATIVE | | COMMERCIAL |
| Format | Fresh | Dry | Dry |
| pH | 3.26 ± 0.07 | 3.16 ± 0.04 | 3.23 ± 0.03 |
| °Brix | 6.9 ± 0.1 | 6.7 ± 0.4 | 6.9 ± 0.1 |
| Degree | 13.4 ± 0 | 12.5 ± 0.5 | 12.8 ± 0.1 |
| Reducing sugars (g/L) | 2.8 ± 0.6 | 2.5 ± 1.7 | 2.7 ± 1.1 |
| EtOH yield (%) | 57.90 ± 0.09 | 54.14 ± 2.22 | 55.21 ± 0.37 |
| Volatile acidity (g/L acetic acid) | 0.29 ± 0.06 | 0.29 ± 0.11 | 0.44 ± 0.02 |
| Total acidity (g/L tartaric acid) | 5.7 ± 0.1 | 6.0 ± 0.2 | 5.7 ± 0.1 |
| Glycerol (g/L) | 6.4 ± 0.1 | 7.4 ± 0.4 | 8.2 ± 0.1 |
| Malic acid (g/L) | 3.07 ± 0.08 | 2.65 ± 0.64 | 2.47 ± 0.01 |
| Colour intensity | 1.380 ± 0.230 | 1.580 ± 0.160 | 1.040 ± 0.100 |
| Tonality | 0.570 ± 0.030 | 0.610 ± 0.040 | 0.650 ± 0.010 |
| TPI | 13 ± 1 | 13 ± 0 | 13 ± 1 |
| Anthocyanins (mg/L) | 85 ± 6 | 84 ± 13 | 84 ± 0 |
| Total tannins (mg/L) | 0.62 ± 0.01 | 0.66 ± 0.01 | 0.65 ± 0.04 |
| CIELAB parameters: | | | |
| L* | 67.370 ± 4.060 | 63.480 ± 2.540 | 73.160 ± 2.300 |
| a* | 43.840 ± 4.990 | 42.460 ± 5.430 | 34.740 ± 0.950 |
| b* | 8.060 ± 0.910 | 8.620 ± 1.460 | 6.780 ± 0.410 |

From the organoleptic standpoint, the tasters described the wines as correct, without sensorial defects which rejected any of the formats of presentation of the native yeast.

### Microvinification II

The experimental design developed in Microvinification I was repeated, using the must indicated as 2 with a °Brix slighter higher (25°Brix) in order to verify the effectiveness of the native yeast in a medium with a greater sugar content.

In all cases, the microvinifications were carried out by inoculation of the native yeast in its two formats (fresh in paste and dry) and the commercial yeast on Must 2.

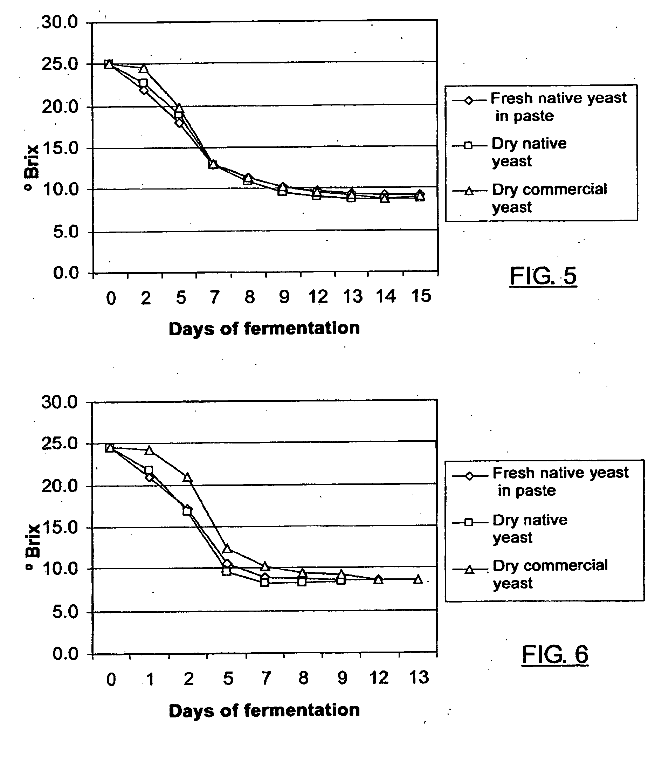

Figure 5 shows the fermentation kinetics of these yeasts expressed as the evolution of the °Brix of the must over time.

As with microvinification I, the native yeast started the fermentation immediately after its addition to the must, whilst the commercial yeast has a greater "adaptation period". All yeasts ended fermentation in 15 days and the resulting wines had similar residual sugar levels.

Table 7 shows the analytical results of the wines obtained from the microvinifications by inoculation of the native yeast in its different formats and the commercial yeast on must 2.

**Table 7. Physicochemical parameters of the wines obtained by inoculation of the native yeast in its different formats and the commercial yeast on must 2 ± S.D.**

| Yeast | NATIVE | | COMMERCIAL |
|---|---|---|---|
| Format | Fresh | Dry | Dry |
| pH | 3.32 ± 0.0 | 3.33 ± 0.04 | 3.31 ± 0.01 |
| °Brix | 9.5 ± 0.1 | 8.7 ± 0.4 | 9.3 ± 0.4 |
| Degree | 13.7 ± 0.3 | 13.9 ± 0.5 | 13.6 ± 0.3 |
| Reducing sugars (g/L) | 21.7 ± 1.6 | 14.0 ± 6.2 | 19.5 ± 4.5 |
| EtOH yield (%) | 44.01 ± 1.08 | 44.55 ± 1.53 | 43.51 ± 0.06 |
| Volatile acidity (g/L acetic acid) | 0.38 ± 0.02 | 0.53 ± 0.02 | 0.36 ± 0.04 |
| Total acidity (g/L tartaric acid) | 6.0 ± 0.1 | 6.1 ± 0.3 | 6.2 ± 0.1 |
| Glycerol (g/L) | 8.6 ± 1.1 | 8.1 ± 0.6 | 8.7 ± 1.1 |
| Malic acid (g/L) | 3.10 ± 011 | 2.84 ± 0.23 | 2.97 ± 0.21 |
| Colour intensity | 1.910 ± 0.980 | 1.240 ± 0.210 | 1.650 ± 0.410 |
| Tonality | 0.750 ± 0.070 | 0.720 ± 0.040 | 0.740 ± 0.070 |
| TPI | 15 ± 1 | 14 ± 0 | 15 ± 1 |
| Anthocyanins (mg/L) | 95 ± 1 | 93 ± 3 | 93 ± 3 |
| Total tannins (mg/L) | 0.73 ± 0.02 | 0.70 ± 0.01 | 0.73 ± 0.01 |
| CIELAB parameters: | | | |
| L* | 57.650 ± 18.680 | 48.680 ± 24.17 | 61.620 ± 8.900 |
| a* | 30.790 ± 7.990 | 32.540 | 32.530 ± 7.370 |
| b* | 7.900 ± 3.170 | 7.230 ± 2.92 | 8.540 ± 2.210 |

The analytical results did not show relevant differences between the different formats of native yeast nor with respect to the commercial yeast. On a tasting level, all wines were described as correct without finding large differences between the wines tested.

### Microvinification III

The objective of the test was to observe the evolution of the strains in a must with solids in suspension and to see its influence on the fermentation kinetics, in the analytical parameters of the wines, as well as on its organoleptic characteristics. Likewise, also to see its interaction with the indigenous yeasts of the must, which in this case grew during fermentation together with the yeast inoculated. The yeasts tested and the formats tested were the same as those tested for previous microvinifications.

Figure 6 shows the fermentation kinetics of these yeasts expressed as the evolution of the °Brix of the must over time.

The fermentations with this must were much faster than in the previous microvinifications, and in only 12 days were finished, since the content of solids in suspension stimulated the growth and metabolism of the yeasts during fermentation. In spontaneous fermentation and in the fermentations with commercial yeasts, the fermentation start-up was slower than in the fermentations inoculated with the native yeast. No significant differences were found in the final Brix degree.

Table 8 shows the analytical results of the wines obtained by inoculation of Must 2 with paste, with the native yeast and the commercial yeast.

**Table 8. Physicochemical parameters of the wines obtained by inoculation of the native yeast in its different formats and the commercial yeast on must 2 ± S.D.**

| Yeast | NATIVE | | COMMERCIAL |
|---|---|---|---|
| Format | Fresh | Dry | Dry |
| pH | 3.57 ± 0.02 | 3.55 | 3.54 |
| °Brix | 8.6 ± 0.0 | 8.3 | 8.6 |
| Degree | 14.5 ± 0.1 | 13.5 | 14 |
| Reducing sugars (g/L) | 1.6 ± 0.1 | 1.4 | 1.5 |
| EtOH yield (%) | 46.58 ± 0.27 | 43.26 | 44.90 |
| Volatile acidity (g/L acetic acid) | 0.24 ± 0.04 | 0.30 | 0.30 |
| Total acidity (g/L tartaric acid) | 6.5 ± 0.1 | 6.8 | 6.4 |
| Glycerol (g/L) | 9.6 ± 0.6 | 10.7 | 12.5 |
| Malic acid (g/L) | 2.95 ± 0.08 | 2.61 | 2.47 |
| Colour intensity | 2.250 ± 0.040 | 2.790 | 1.940 |
| Tonality | 0.860 ± 0.010 | 0.790 | 0.870 |
| TPI | 18 ± 3 | 20 | 16 |
| Anthocyanins (mg/L) | 131 ± 1 | 140 | 104 |
| Total tannins (mg/L) | 0.94 ± 0.14 | 0.98 | 0.80 |
| CIELAB parameters: | | | |
| L* | 55.070 ± 1.480 | 49.300 | 60.510 |
| a* | 50.530 ± 3.490 | 57.800 | 50.330 |
| b* | 17.520 ± 5.060 | 18.430 | 12.530 |

As with the previous microvinifications, no significant differences were found in the values obtained with the parameters analysed between the native yeast in its different formats with respect to the commercial yeast.

In the tasting panel, the wines produced with the native yeast have been evaluated independently from the tasted yeast format.

### Microvinification IV

In this case, the microvinification was performed using Must 1 as a base, with a somewhat higher average sugar concentration level in the must (23 ° Brix).

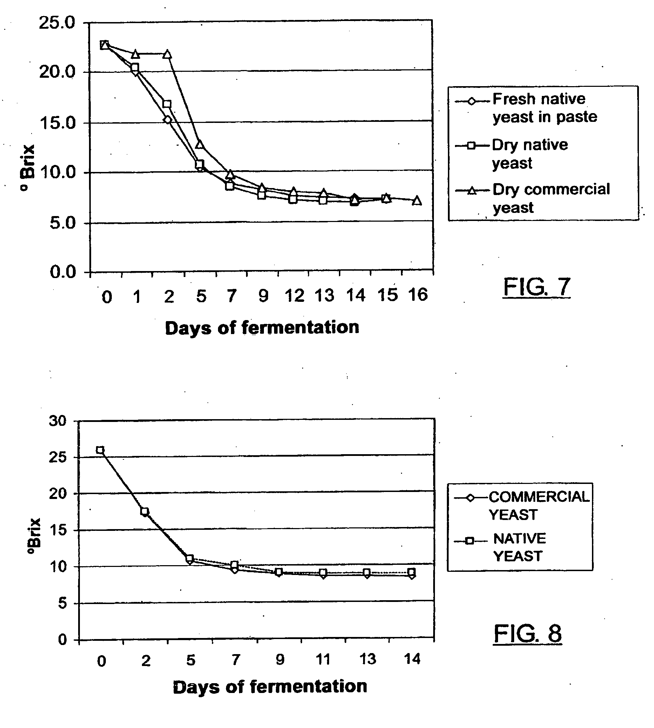

Figure 7 shows the fermentation kinetics of these yeasts expressed as the evolution of the °Brix of the must over time.

The fermentations ended after 16 days and the wines were removed to do the chemical analysis and testing. In this case, the wines inoculated with the dry native and commercial yeast took a little longer than the others to start fermenting. However, all finished in a similar time, and with a similar residual Brix degree.

The following table shows the analytical results of the wines obtained by inoculation of Must 1 with greater sugar content with the native yeast and the commercial yeast.

**Table 9. Physicochemical parameters of the wines obtained by inoculation of the native yeast in its different formats and the commercial yeast on must 1 with greater sugar content. Average ± S.D.**

| Yeast | NATIVE | | COMMERCIAL |
|---|---|---|---|
| Format | Fresh | Dry | Dry |
| pH | 3.13 ± 0.17 | 3.20 ± 0.07 | 3.01 ± 0.18 |
| °Brix | 7.4 ± 0.3 | 6.9 ± 0.4 | 7.2 ± 0.0 |
| Degree | 12.7 ± 0.3 | 12.6 ± 0.6 | 12.6 ± 0.8 |
| Reducing sugars (g/L) | 5.4 ± 4.6 | 2.4 ± 2.5 | 3.9 ± 0.4 |
| EtOH yield (%) | 54.67 ± 1.33 | 54.39 ± 2.44 | 54.50 ± 3.53 |
| Volatile acidity (g/L acetic acid) | 0.35 ± 0.06 | 0.47 ± 0.11 | 0.32 ± 0.02 |
| Total acidity (g/L tartaric acid) | 6.0 ± 0.3 | 5.5 ± 0.1 | 6.3 ± 0.1 |
| Glycerol (g/L) | 7.6 ± 0.1 | 7.6 ± 0.6 | 8.9 ± 0.5 |
| Malic acid (g/L) | 2.31 ± 0.03 | 2.39 ± 0.24 | 2.10 ± 0.01 |
| Colour intensity | 1.480 ± 0.120 | 1.750 ± 0.410 | 1.550 ± 0.020 |
| Tonality | 0.540 ± 0.000 | 0.600 ± 0.040 | 0.530 ± 0.010 |
| TPI | 12 ± 0 | 12 ± 0 | 12 ± 0 |
| Anthocyanins (mg/L) | 137 ± 3 | 120 ± 14 | 130 ± 4 |
| Total tannins (mg/L) | 0.62 ± 0.01 | 0.61 ± 0.01 | 0.61 ± 0.01 |

| CIELAB parameters: | | | |
|---|---|---|---|
| L* | 65.020 ± 2.330 | 58.930 ± 7.250 | 63.730 ± 0.280 |
| a* | 45.790 ± 0.080 | 43.760 ± 4.760 | 47. 290 ± 1.170 |
| b* | 5.490 ± 0.090 | 6.410 ± 0.270 | 6.400 ± 0.110 |

The results obtained with the microvinifications carried out by inoculation of the native yeast in its two formats (fresh in paste, dry) and the commercial yeast show that:
- The native yeast, both with different musts and in its different formats, showed a high fermenting capacity, the fermentation ending in 23 °Brix musts, corresponding to a high probable alcohol degree of 13.5%.
- Both in musts with 23 °Brix and with 25 °Brix, the native yeast began fermentation more vigorously. The commercial yeast had a slower fermentation start.
- None of the yeasts tested formed high quantities of acetic acid and the volatile acidity values were below 0.54 g/L in all microvinifications.
- The total acidity was always under 6.7 g of tartaric acid/L.
- Glycerol formation by the native yeasts was similar to the commercial strain, characterized by a high production of this compound. The format type the yeast was presented in did not influence glycerol production.
- In all fermentations, the native yeast gave rise to wines with similar degrees of alcohol to those produced with commercial yeast.
- In terms of ethanol yield, there was significant differences between the commercial and native yeast.
- With respect to contribution of the native yeast to the wine colour parameters, this was conditional upon the type of must fermented. Within each type of must, there was no differences between the yeasts inoculated and the different formats tested.
- With respect to the sensorial analysis of the wines tested, the native yeast in all its formats had a similar behaviour to the commercial yeast in all tests.

### Example 4: Industrial fermentations

The yeast GBiot-EL 011 (CECT 13030), tested in the microvinification tests on laboratory scale, was tested during three consecutive wine harvests in two wineries of Rioja Alavesa (Bodegas Vinos de los Herederos de Marqués de Riscal and Bodegas Baigorri). In addition to this yeast, the same commercial yeast as used in the previous examples was used as control. The two yeasts were tested in tanks containing Tempranillo grape must.

The native yeast was supplied to the wineries in fresh format in liquid and in paste. The commercial yeast was tested in the form of active dry yeast, which is the commercially available form.

### Industrial scale fermentation with the liquid fresh format

The native yeast was tested in the two aforementioned wineries of Rioja Alavesa during two consecutive wine harvests. The yeast was supplied in liquid format. The inoculations were performed in tanks with 20,000 kg of grapes. The grape variety on which the inoculation was performed was the Tempranillo variety.

### 2006 wine harvest inoculation

Table 10 shows the analytical parameters of the must (before inoculation) and the wine after alcoholic fermentation (FOH), in winery 1 of the tank inoculated with the commercial yeast of the tank inoculated with the native yeast in its liquid format.

| | COMMERCIAL YEAST | | NATIVE YEAST IN LIQUID FORMAT | |
|---|---|---|---|---|
| | BEFORE | AFTER | BEFORE | AFTER |
| | INOCULATING | FOH | INOCULATING | FOH |
| pH | 3.5 | 3.52 | 3.52 | 3.62 |
| °Brix | 23.6 | 7.8 | 22.2 | 7.6 |
| Degree (v/v) | 0.00 | 13.46 | 0.00 | 13.45 |
| EtOH yield (%) | | | | |
| Reducing sugars (g/L) | 251 | 6 | 247 | 3 |
| Free SO₂ (mg/L) | 16 | 0 | 0 | 0 |
| Combined SO₂ (mg/L) | 17 | 7 | 32 | 13 |
| Total SO₂ (mg/L) | 33 | 7 | 32 | 13 |
| Volatile acidity (g/L acetic acid) | 0.09 | 0.24 | 0.21 | 0.39 |
| Volatile acidity (g/L tartaric acid) | 4.5 | 5.9 | 4.5 | 5.2 |
| Glycerol (g/L) | 0.1 | 8.7 | 0.3 | 7.8 |
| Malic acid (g/L) | 3.11 | 3.12 | 2.22 | 2.64 |
| Colour intensity | 5.458 | 8.628 | 3.751 | 9.389 |
| Tonality | 0.416 | 0.544 | 0.512 | 0.588 |
| TPI | 26 | 42 | 15 | 50 |
| Anthocyanins (mg/L) | 551 | 619 | 218 | 647 |
| Total tannins (mg/L) | 1.3 | 2.1 | 0.8 | 2.5 |
| CIELAB parameters: | | | | |
| L* | 30.005 | 17.594 | 36.909 | 15.631 |
| a* | 60.168 | 48.448 | 58.76 | 47.997 |
| b* | 23.639 | 28.414 | 13.075 | 26.246 |

Table 11 shows the analytical parameters of the must (before inoculating) and the wine after alcoholic fermentation (FOH), of winery 2 of one of the fermentation tanks inoculated with the commercial yeast with the native yeast in fresh liquid format.

| | COMMERCIAL YEAST | | NATIVE YEAST IN LIQUID FORMAT | |
|---|---|---|---|---|
| | BEFORE INOCULATING | AFTER FOH | BEFORE INOCULATING | AFTER FOH |
| pH | No data | 3.62 | 3.69 | 3.8 |
| °Brix | No data | 8 | 25.2 | 8.8 |
| Degree (v/v) | No data | 13.46 | 0 | 14.43 |
| EtOH yield (%) | No data | | | |
| Reducing sugars (g/L) | No data | 1 | 273 | 2 |
| Free SO₂ (mg/L) | No data | 2 | 3 | 6 |
| Combined SO₂ (mg/L) | No data | 8 3 | | 15 |
| Total SO₂ (mg/L) | No data | 10 | 6 | 21 |
| Volatile acidity (g/L acetic acid) | No data | 0.33 | 0.06 | 0.39 |
| Volatile acidity (g/L tartaric acid) | No data | 5.6 | 4.4 | 6.1 |
| Glycerol (g/L) | No data | 5.8 | 0.3 | 9.5 |
| Malic acid (g/L) | No data | 2.06 | 3.57 | 3.43 |
| Colour intensity | No data | 12.27 | 3.459 | 11.12 |
| Tonality | No data | 0.535 | 0.692 | 0.591 |
| TPI | No data | 52 | 18 | 54 |
| Anthocyanins (mg/L) | No data | 749 | 212 | 902 |
| Total tannins (mg/L) | No data | 2.6 | 0.9 | 2.7 |
| CIELAB parameters: | | | | |
| L* | No data | 12.491 | 39.953 | 11.125 |
| a* | No data | 43.633 | 53.025 | 41.424 |
| b* | No data | 21.415 | 20.756 | 18.909 |

With respect to implantation of the native yeast, good implantation results were observed in both wineries.

The results showed exceptional physicochemical characteristics in the wine produced by inoculation of the must with the native yeast selected, comparatively with the wine produced by inoculation of the must with the commercial yeast usually used in the winery. On a tasting level, the wines obtained were classified by the enologists of the wineries as very correct and good quality wines.

All these results reveal the exceptional characteristics of the native yeast isolated and selected from Rioja Alavesa, for the production of extraordinary quality wine and which maintains the typical characteristics of the wines of this important wine region.

### 2007 wine harvest inoculation with fresh format in paste

The inoculations were carried out in tanks holding 20,000 kg of grapes belonging to the same wineries as above. The grape variety on which the inoculation was carried out was yeast of the Tempranillo variety. In this wine harvest, the native yeast was tested in the form of paste. As with the previous wine harvests, the same commercial yeast was used as control.

Table 14 shows the data obtained in the course of the fermentation of the tanks inoculated with the fresh yeast in paste and they were compared with the details for the inoculations with the commercial yeast typically used in the winery. Vinification in winery 1

| | **Fresh native yeast in paste** | | **Commercial yeast** | |
|---|---|---|---|---|
| | BEFORE INOCULATING | AFTER FOH | BEFORE INOCULATING | AFTER FOH |
| pH | 3.41 | 3.55 | 3.31 | 3.56 |
| °Brix | 23.2 | 8 | 23.2 | 7.6 |
| Degree (v/v) | 0 | 13.94 | 0 | 13.46 |
| EtOH yield (%) | 0 | 49.61 | 0 | 48.11 |
| Reducing sugars (g/L) | 222 | 6 | 221 | 5 |
| Free SO₂ (mg/L) | 0 | 0 | 10 | 0 |
| Combined SO₂ (mg/L) | 15 | 5 | 23 | 0 |
| Total SO₂ (mg/L) | 15 | 5 | 33 | 0 |
| Volatile acidity (g/L acetic acid) | 0.24 | 0.21 | 0.09 | 0.18 |
| Volatile acidity (g/L tartaric acid) | 6.4 | 5.8 | 6.9 | 6.1 |
| Glycerol (g/L) | 0.9 | 8.7 | 0.2 | 8.1 |
| Malic acid (g/L) | 4.38 | 3.58 | 4.56 | 3.82 |
| Colour intensity | 8.841 | 10.151 | 6.89 | 8.148 |
| Tonality | 0.43 | 0.515 | 0.373 | 0.493 |
| TPI | 24 | 53 | 19 | 48 |
| Anthocyanins (mg/L) | 351 | 502 | 364 | 489 |
| Total tannins (mg/L) | 1.2 | 2.6 | 1 | 2.4 |
| CIELAB parameters: | | | | |
| L* | 20.128 | 16.278 | 30.27 | 23.614 |
| a* | 50.441 | 47.695 | 60.729 | 56.223 |
| b* | 31.955 | 27.293 | 39.1 | 37.178 |

The results show very good physicochemical characteristics in the wine produced by inoculation of the must with this native yeast selected, comparatively with the use of the commercial yeast.

The alcoholic and malolactic fermentations of the tanks on which the native yeasts had been inoculated took place with complete normality, producing the wines perfectly. The fermentations with this yeast took place with total normality, producing very high quality wines and with organoleptic evaluations by tasting which were exceptional, according to the details collected by the participating wineries.

The results obtained with the industrial fermentations carried out with the native yeast in its fresh format in fresh paste and commercial yeast allow us to draw the following general conclusions:
- The native yeast has good implantation results.
- The native yeast in its fresh format in paste has shown a high fermenting capacity and has given rise to wines with a suitable alcohol degree for Rioja wines. Good yield was also reached in ethanol production with both formats of native yeast.
- The wines produced with fresh native yeast in paste have a volatile acidity lower than 0.35 g/L after alcoholic fermentation and total acidity lower than 6.8 g of tartaric acid/L. These values are suitable for Rioja wines.
- Inoculation with fresh native yeast in paste gave rise to wines with high glycerol content, even higher values than with the commercial yeast.
- It can be concluded according to the results obtained as a whole, that the fresh native yeast in paste has a high technological quality.

### Vinification in winery 2

Table 15 shows the analytical results during the course of fermentation in winery 2 of the tanks where the fresh native yeast in paste has been inoculated and they were compared with the data from the commercial yeast typically used in the winery.

| | **Fresh native yeast in paste** | | **Commercial yeast** | |
|---|---|---|---|---|
| | BEFORE INOCULATING | AFTER FOH | BEFORE INOCULATING | AFTER FOH |
| pH | 3.3 | 3.41 | 3.22 | 3.52 |
| °Brix | 23.6 | 7.4 | 22.4 | 7.6 |
| Degree (v/v) | 0 | 13.46 | 0 | 13.46 |
| EtOH yield (%) | 0 | 51.37 | 0 | 54.81 |
| Reducing sugars (g/L) | 207 | 1 | 194 | 2 |
| Free SO₂ (mg/L) | 0 | 0 | 0 | 0 |
| Combined SO₂ (mg/L) | 0 | 0 | 0 | 22 |
| Total SO₂ (mg/L) | 0 | 0 | 0 | 22 |
| Volatile acidity (g/L acetic acid) | 0 | 0.21 | 0.03 | 0.27 |
| Volatile acidity (g/L tartaric acid) | 5.2 | 5.8 | 5.8 | 5.6 |
| Glycerol (g/L) | 0.3 | 9.1 | 0.4 | 8.8 |
| Malic acid (g/L) | 3.69 | 2.9 | 3.03 | 3.15 |
| Colour intensity | 2.588 | 8.801 | 3.69 | 8.064 |
| Tonality | 0.71 | 0.491 | 0.633 | 0.552 |
| TPI | 13 | 44 | 18 | 49 |
| Anthocyanins (mg/L) | 93 | 552 | 140 | 670 |
| Total tannins (mg/L) | 0.6 | 2.2 | 0.9 | 2.4 |

| CIELAB parameters: | | | | |
|---|---|---|---|---|
| L* | 47.792 | 20.529 | 39.386 | 17.634 |
| a* | 48.681 | 52.12 | 55.19 | 47.498 |
| b* | 18.96 | 33.092 | 23.745 | 26.845 |

The results show exceptional physicochemical characteristics in the wine produced by inoculation of the must with the native yeast selected comparatively with the wine produced by inoculation of the must with the commercial yeast typically used in the winery.

All these results reveal the exceptional characteristics of the native yeast isolated and selected from Rioja Alavesa, for the production of extraordinary quality wine and which maintains the typical characteristics of the wines of this important wine region.

In addition to evaluating the contribution of the yeasts from a physicochemical standpoint, the capacity of implanting in the inoculated tanks compared with the indigenous population present in the must was studied. The study of implantation combined the molecular mitochondrial DNA technique and determination of the *killer* phenotype. The results obtained for each one of the wineries are shown below.

The results obtained with the industrial fermentations carried out with the native yeast in its fresh format in fresh paste and commercial yeast show that:
- The fresh native yeast in paste has shown good implantation results.
- The native yeast in its fresh format in paste has shown a high fermenting capacity and has given rise to wines with a suitable alcohol degree for Rioja wines. Good yield was also reached in ethanol production.
- The wines produced with fresh native yeast in paste have a volatile acidity lower than 0.25 g/L after alcoholic fermentation and total acidity lower than 6.0 g of tartaric acid/L. These values are suitable for Rioja wines.
- Inoculation with fresh native yeast in paste gave rise to wines with high glycerol content, even higher values than with the commercial yeast.
- The fresh yeast in paste gave rise to a wine with a high colour intensity and high robe after alcoholic fermentation.

### Example 5: Differentiation of native yeast as compared with commercial yeast.

In order to verify if these are differences in fructose consumption between the native yeast strain isolated from grapes from the vineyards of Rioja Alavesa and the reference commercial yeast, a series of microvinification processes were carried out in the laboratory, inoculating each one of the yeasts in different fermentation flasks which contained sterile must of the Tempranillo variety, from which the initial glucose and fructose content were determined. The size of the inoculation both of the native yeast and commercial yeast, with which the sterile yeast was inoculated was of 2 x 10⁸ CFU/mL.

Table 16 indicates the characteristics of the sterile must used for the microvinifications.

**Table 16**

| ANALYTICAL PARAMETERS | MUST |
|---|---|
| pH | 3.53 |
| °Brix | 26 |
| YAN (mg/L) | 249.2 |
| Glucose (g/L) | 155.81 |
| Fructose (g/L) | 164.51 |

### Microvinification I

The following figure represents the fermentation kinetics of the yeasts expressed as the evolution of the °Brix of the grape must over time.

Figure 8 shows that both the native yeast and the commercial strain showed fast and regular fermentation kinetics and ended fermentation in 14 days.

The analytical results of the wines produced from the inoculated must are shown in table 17.

**Table 17**

| SAMPLE | COMMERCIAL YEAST | NATIVE YEAST |
|---|---|---|
| pH | 3.5 | 3.6 |
| °Brix | 8.8 | 8.4 |
| YAN (mg/L) | 78.4 | 78.4 |
| Glucose (g/L) | 0.316 | 0.215 |
| Fructose (g/L) | 7.625 | 3.921 |

### Microvinification II

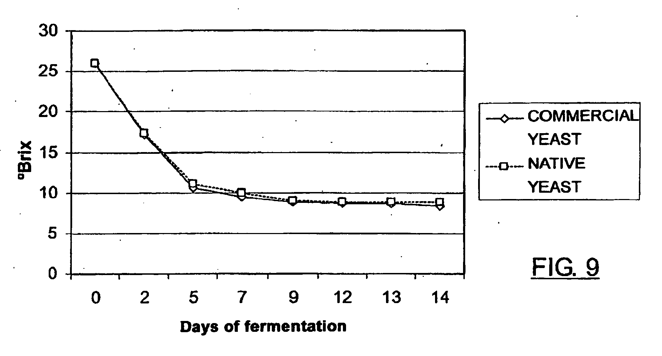

Figure 9 represents the fermentation kinetics of the yeasts expressed as the evolution of the °Brix of the grape must over time.

Both the native yeast and the commercial strain showed fast and regular fermentation kinetics and ended fermentation in 14 days.

The analytical results of the wines produced in microvinification II are shown in table 18.

| SAMPLE | COMMERCIAL YEAST | NATIVE YEAST |
|---|---|---|
| pH | 3.48 | 3.51 |
| °Brix | 9 | 8.6 |
| YAN (mg/L) | 78.4 | 78.4 |
| Glucose (g/L) | 0.65 | 0.189 |
| Fructose (g/L) | 11.212 | 6.119 |

From the data obtained in these two microvinifications it is observed that there is a considerable difference with respect to fructose consumption between the two yeasts. Both in microvinification I and II, the native yeast consumed more fructose than the commercial, the value obtained of the residual fructose in the must inoculated with the native yeast being in the order of half that obtained with the commercial yeast. It is also observed, although with a less significant difference, that the native yeast made it possible to obtain a wine with less glucose content.

These data show that the native yeast had a greater consumption capacity and, therefore, greater fructose assimilation of the must fructose, compared to commercial yeast.

## Claims

1. Strain of the species *Saccharomyces cerevisiae* identified as GBiot-EL 011 deposited in the Spanish Type Culture Collection (CECT), with access number CECT 13030.

2. Strain according to claim 1, **characterized in that** it is in liquid format, fresh in paste, active dry or instant dry.

3. Use of the strain of the species *Saccharomyces cerevisiae* identified as GBiot-EL 011 deposited in the CECT with access number CECT 13030 in the production of alcoholic drinks produced from alcoholic fermentation.

4. Use according to claim 3, where the alcoholic drink is selected from wine, beer, cava and cider.

5. Use according either of claims 3-4, where the alcoholic drink is wine produced from grape must.

6. Use according to claim 5, where the grape is of the Tempranillo variety.

7. Use of the strain of the species *Saccharomyces cerevisiae* identified as GBiot-EL 011 deposited in the CECT with access number CECT 13030 for biomass production.

## Patentansprüche

1. Stamm der Spezies *Saccharomyces cerevisiae,* der als GBiot-EL 011 identifiziert und in der spanischen Sammlung von Mikroorganismen und Zellkulturen (CECT) unter der Zugangsnummer CECT 13030 hinterlegt ist.

2. Stamm nach Anspruch 1, **dadurch gekennzeichnet, dass** er in Flüssigform, Frischpaste, aktiv trocken oder sofort trocken vorliegt.

3. Verwendung des Stammes der Spezies *Saccharomyces cerevisiae,* der als GBiot-EL 011 identifiziert und in der CECT unter der Zugangsnummer CECT 13030 hinterlegt ist, zur Herstellung alkoholischer Getränke, die durch alkoholische Gärung hergestellt werden.

4. Verwendung nach Anspruch 3, wobei das alkoholische Getränk aus Wein, Bier, Sekt und Apfelwein ausgewählt wird.

5. Verwendung nach einem der Ansprüche 3 bis 4, wobei das alkoholische Getränk aus Traubenmost hergestellter Wein ist.

6. Verwendung nach Anspruch 5, wobei die Traube von der Sorte Tempranillo ist.

7. Verwendung des Stammes der Spezies *Saccharomyces cerevisiae,* der als GBiot-EL 011 identifiziert und in der CECT unter der Zugangsnummer CECT 13030 hinterlegt ist, zur Herstellung von Biomasse.

## Revendications

1. Souche de l'espèce *Saccharomyces cerevisiae* identifiée comme GBiot-EL 011 déposée dans la Collection de cultures de type espagnol (CECT), sous le numéro d'accès CECT 13030.

2. Souche selon la revendication 1, **caractérisée en ce qu'**elle est sous forme liquide, fraîche dans la pâte, sèche active ou sèche instantanée.

3. Utilisation de la souche de l'espèce *Saccharomyces cerevisiae* identifiée comme GBiot-EL 011 déposée dans la CECT, sous le numéro d'accès CECT 13030 dans la production de boissons alcooliques issues de la fermentation alcoolique

4. Utilisation selon la revendication 3, où la boisson alcoolique est choisi parmi vin, bière, cava et cidre.

5. Utilisation selon l'une des revendications 3-4, où la boisson alcoolisée est le vin produit à partir du moût de raisin.

6. Utilisation selon la revendication 5, où le raisin est de la variété Tempranillo.

7. Utilisation de la souche de l'espèce *Saccharomyces cerevisiae* identifiée comme GBiot-EL 011 déposée dans la CECT sous le numéro d'accès CECT 13030 pour la production de biomasse.
